# FASCICULE DE BREVET EUROPEEN

(11) **EP 3 512 473 B1**
(45) Date de publication et mention de la délivrance du brevet: **30.09.2020**
(21) Numéro de dépôt: 17784341.4
(22) Date de dépôt: 12.09.2017
(51) Int. Cl.: A61F 7/00, A61F 7/12, H01S 3/04

(54) **DISPOSITIF DE REFROIDISSEMENT LOCALISÉ D'UN ORGANE**
VORRICHTUNG ZUR ÖRTLICH BEGRENZTEN KÜHLUNG EINES ORGANS
DEVICE FOR LOCALIZED COOLING OF AN ORGAN

(30) Priorité: 15.09.2016 FR 1658654
(43) Date de publication de la demande: 24.07.2019
(73) Titulaire: Commissariat à l'énergie atomique et aux énergies alternatives, 75015 Paris (FR); Université Grenoble Alpes, 38400 Saint-Martin-d'Hères (FR); Centre Hospitalier Universitaire Grenoble Alpes, 38700 La Tronche (FR)
(72) Inventeur: AUBERT, Nicolas, 38000 Grenoble (FR); CHABROL, Claude, 38320 Poisat (FR); CHABARDES, Stephan, 38610 Venon (FR); DURAFFOURG, Laurent, 38500 Voiron (FR)
(74) Mandataire: GIE Innovation Competence Group
(86) Numéro de dépôt international: PCT/FR2017/052423
(87) Numéro de publication internationale: WO 2018/051005

(56) Documents cités:
- WO-A1-2016/102351
- JP-A- 2006 015 064
- US-A- 5 620 571
- US-A1- 2007 005 121
- US-B1- 9 362 712

## Description

### Domaine technique de l'invention

La présente invention se rapporte à un dispositif de refroidissement localisé d'un organe, tel que par exemple le cerveau humain.

### Etat de la technique

Il est connu de traiter certaines maladies en effectuant un refroidissement localisé des tissus. C'est le cas par exemple des maladies comme l'épilepsie dans lesquelles un refroidissement localisé de la zone épileptogène permet de bloquer l'émergence de crises ou de limiter leur propagation. Ceci présente un intérêt pour les patients atteints de cette pathologie et que l'on ne peut traiter, du fait d'un caractère phamaco-résistant, par les médicaments classiques. Cependant, les dispositifs de refroidissement sont souvent peu adaptés à une utilisation en implantation chronique intra cérébrale. Certaines solutions connues, non adaptées à l'implantation, sont par exemple basées sur des principes microfluidiques et utilisent des modules à effet Peltier.

Différentes solutions sont décrites dans les documents référencés US2007/005121A1**,** JP2006/015064A**,** US5620571A**,** US9362712B1 et WO2016/102351A1**.**

Le but de l'invention est ainsi de proposer un dispositif de refroidissement qui présente une configuration adaptée à une implantation longue durée, qui minimise les risques médicaux, que ce soit lors de son implantation ou en fonctionnement et qui peut fonctionner à tout instant sur commande, en boucle fermée ("closed loop"), par exemple lors de l'apparition d'une crise d'épilepsie.

### Exposé de l'invention

Ce but est atteint par un dispositif de refroidissement localisé d'un organe, comportant :
- Une tige de forme allongée comportant une extrémité distale destinée à venir en contact avec un organe à refroidir et comportant :
   o Un élément de refroidissement comportant un doigt froid,
   ∘ Un cristal à capacité de refroidissement par excitation à une longueur d'onde d'excitation déterminée, positionné de manière adjacente audit élément de refroidissement,
   ∘ Un guide optique apte à véhiculer un signal lumineux à ladite longueur d'onde d'excitation et débouchant sur ledit cristal,
- Un système d'illumination comportant au moins une source de lumière agencée pour émettre ledit signal lumineux.

Selon une particularité, le dispositif comporte une cavité dans laquelle est logé ledit cristal. L'élément de refroidissement est préférentiellement positionné à proximité de ladite cavité de manière à assurer le transfert thermique entre le cristal refroidissant et son doigt froid.

Selon une autre particularité, l'élément de refroidissement comporte un échangeur thermique positionné entre le doigt froid et la cavité enfermant le cristal.

Selon une autre particularité, le dispositif comporte au moins un deuxième guide optique agencé le long la tige et débouchant sur ledit cristal de manière à évacuer ou surveiller le niveau de fluorescence émis par l'excitation du cristal.

Avantageusement, le dispositif comporte une unité de commande et de traitement agencée pour commander ledit système d'illumination.

Avantageusement, il comporte un capteur de température agencé pour déterminer la température du cristal.

Selon une particularité, ledit capteur de température comporte des moyens de détermination de la température du cristal à partir de la longueur d'onde du signal lumineux émis par la source de lumière.

Avantageusement, la tige est formée d'un surmoulage en silicone ou polyuréthane.

Préférentiellement, le cristal est de type Yb:YLF, Zb:LANP ou Tm:Yb:YLF. Avantageusement, la source de lumière du dispositif est choisie parmi :
- une diode Laser,
- un Laser à fibre,
- un Laser de type DPSS ("Diode-Pumped Solid State"), ou
- un Laser de type OPS ("Optically-Pumped Semiconductor").

Le dispositif de l'invention pourra notamment être intégré dans un système fonctionnant en boucle fermée et employant des moyens de détection adaptés. Les moyens de détection sont par exemple agencés pour détecter un foyer épileptogène et le dispositif de l'invention est alors adapté pour générer un refroidissement d'intensité adaptée pour contribuer alors à l'arrêt de la crise ou à bloquer son émergence.

### Brève description des figures

D'autres caractéristiques et avantages vont apparaître dans la description détaillée qui suit faite en regard des dessins annexés dans lesquels :
- La figure 1 représente, de manière schématique, l'architecture du dispositif de refroidissement de l'invention.
- La figure 2A représente, en détails et de manière schématique, l'architecture de l'extrémité distale du dispositif de refroidissement de l'invention, selon un premier mode de réalisation.
- La figure 2B représente, en détails et de manière schématique, l'architecture de l'extrémité distale du dispositif de refroidissement de l'invention, selon un deuxième mode de réalisation.
- La figure 3 illustre le principe de fonctionnement du cristal refroidissant.
- La figure 4 illustre le principe de fonctionnement d'un capteur pour estimer la température du cristal à partir de la longueur d'onde de la lumière émise.

### Description détaillée d'au moins un mode de réalisation

L'invention concerne un dispositif de refroidissement localisé d'un organe en vue de refroidir ses tissus. Ledit organe sera par exemple le cerveau humain.

De manière non limitative, le dispositif de l'invention sera par exemple adapté au traitement de différentes pathologies, par exemple :
- Crise d'épilepsie,
- Traumatisme crânio-encéphalique,
- Neuro-Cancer,
- Maladie de Parkinson ou autres maladies du mouvement telles que les dystonies, les tremblements essentiels, la chorée de huntington.

Bien entendu, moyennant certaines adaptations, il faut comprendre qu'il pourra être employé pour traiter d'autres pathologies.

Dans le cas du traitement d'une crise d'épilepsie, la source de froid générée est destinée à être mise en contact des foyers épileptogènes ou de toute autre zone du cerveau 30 accessible chirurgicalement en utilisant des méthodes mini invasives stéréotaxiques au travers d'une perforation ("drill") crânienne de quelques millimètres. Le refroidissement généré contribue alors à l'arrêt de la crise ou à bloquer son émergence.

L'invention se présente sous la forme d'un dispositif de refroidissement 1 localisé, biocompatible et implantable de manière à pouvoir agir sur commande à tout instant, par exemple lorsque l'apparition d'une crise d'épilepsie est détectée, en utilisant des algorithmes de détection des crises en boucle fermée ("closed loop").

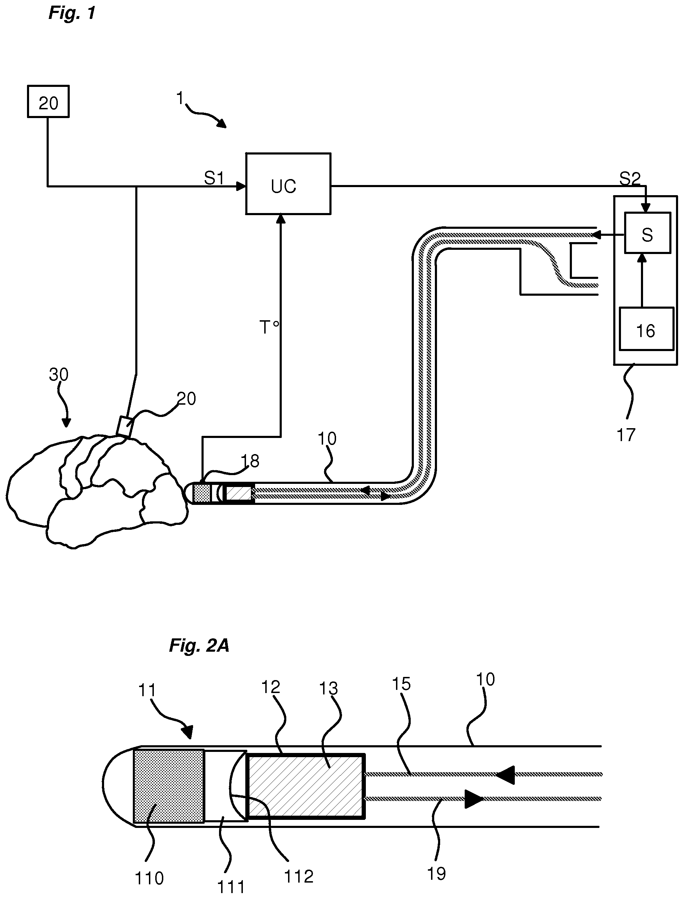

Selon l'application visée, des moyens de détection 20, ne faisant pas l'objet de la présente demande, sont par exemple employés pour détecter l'apparition de la pathologie à traiter. Dans le cas d'une crise d'épilepsie, ces moyens de détection 20 sont implantés au niveau crânien pour détecter une zone épileptogène (figure 1). Lorsque l'apparition d'une crise est détectée, les moyens de détection 20 envoient un signal S1 à une unité de commande et de traitement UC. L'unité de commande et de traitement UC émet alors une commande (S2) vers le dispositif de refroidissement de l'invention. Le dispositif de refroidissement de l'invention est commandé pour générer un refroidissement adapté à la pathologie traitée. S'il s'agit d'une crise d'épilepsie, l'intensité du refroidissement et sa durée d'application seront préférentiellement liés au niveau d'intensité de la crise qui a été mesuré par les moyens de détection 20. Pour générer une commande adaptée vers le dispositif de refroidissement, l'unité de commande et de traitement UC comporte préférentiellement un module d'analyse destiné à analyser le signal S1 reçu en provenance des moyens de détection et à déterminer un traitement adapté.

L'unité de commande et de traitement UC fait avantageusement partie du dispositif de refroidissement 1 de l'invention. Elle comporte au moins un microprocesseur et des moyens de mémorisation. Elle est destinée à exécuter des instructions logicielles représentatives d'une séquence de traitement de la pathologie par le dispositif. Elle comporte notamment des moyens de commande d'un système d'illumination qui sera détaillé ci-dessous. Elle comporte également une ou plusieurs interfaces de communication destinées à communiquer avec les différentes entités, notamment les moyens de détection décrits ci-dessus. Les liaisons de communication pourront être filaire ou sans-fil.

Le dispositif de refroidissement 1 de l'invention se présente sous la forme d'au moins une tige 10 allongée en matériau souple comportant une extrémité distale destinée à venir en contact avec l'organe à refroidir. La section de la tige 10 souple est préférentiellement circulaire. La surface de la section de la tige est suffisamment faible pour être facilement implantable lors d'une opération classique de chirurgie. Dans le cas, d'une tige de section circulaire, son diamètre est compris entre 0.5 et 3 mm, avantageusement entre 1 et 2.5 mm. A son extrémité proximale, la tige 10 comporte avantageusement une connectique de nature optique pour être reliée à une source de lumière et de nature électrique pour être reliée à l'unité de commande et de traitement.

Bien entendu, le dispositif de l'invention est réalisé à partir de matériaux biocompatibles afin de pouvoir être implanté dans un être vivant.

La tige 10 est formée par un surmoulage en matériau souple qui intègre les différents éléments qui sont décrits ci-dessous. Ces éléments sont donc avantageusement enrobés dans le surmoulage, réalisé par exemple en silicone ou en polyuréthane. Ce surmoulage permet notamment d'éviter toute fuite de liquides physiologiques.

Le dispositif 1 comporte ainsi un élément de refroidissement 11 comportant un doigt froid 110 situé à l'extrémité distale de la tige 10 et destiné à venir en contact avec l'organe à refroidir (cerveau 30 sur la figure 1).

Le dispositif 1 comporte une cavité 12 intégrée à la tige et un cristal 13 refroidissant logé dans ladite cavité 12. Le cristal 13 est placé dans la cavité et fixé à cette dernière par des matériaux n'absorbant pas la lumière produite et faiblement conducteur de chaleur. L'élément de refroidissement 11 est destiné à capter la variation de température négative générée par le cristal refroidissant lorsque ce dernier est excité de manière adaptée. L'élément de refroidissement est préférentiellement positionné au plus proche de ladite cavité 12 pour un meilleur transfert thermique entre les parois de ladite cavité et lui-même.

Le cristal 13 refroidissant est préférentiellement formé d'un matériau adapté pour fonctionner selon un principe dit de "fluorescence anti-Stokes". Ce principe consiste en une diffusion inélastique de la lumière, impliquant un échange d'énergie entre un photon incident d'un signal lumineux venant frapper le cristal à une longueur d'onde déterminée et le cristal. La lumière diffusée par le cristal n'a ainsi pas la même longueur d'onde que la lumière incidente. Dans le cas d'un décalage anti-Stokes, la lumière diffusée présente une longueur d'onde plus faible que la lumière incidente mais avec une énergie plus importante, ce qui aboutit au refroidissement du cristal.

De manière non limitative, le cristal employé sera préférentiellement formé de toute matrice hôte dopée à l'ytterbium à basse énergie de phonons. Il s'agira par exemple d'un cristal de type Yb: YLF. Sa longueur d'onde d'excitation est comprise entre 1010nm et 1040 nm environ.

Bien entendu, toute autre composition de cristal pourra être envisagée comme par exemple celle d'un cristal de YLF co-dopé 5% Yb - 0.0016% Tm. Une attention particulière sera apportée à la pureté des cristaux utilisés. On utilisera par exemple des composants de pureté 5N lors de la fabrication de la matrice hôte.

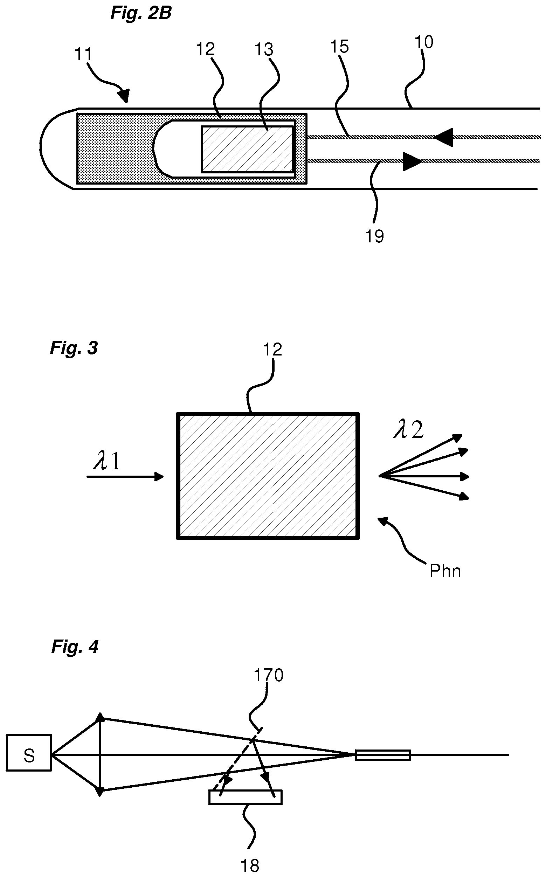

Dans le cas d'un cristal dopé avec de l'ytterbium, le schéma de refroidissement est le suivant : soit un électron initialement sur le niveau E4. Cet électron vient être porté à un état excité E5 par l'absorption d'un photon incident de longueur d'onde 1020 nm (illustré par la longueur d'onde d'excitation λ1 sur la figure 3). Il est ensuite porté à l'état E6 par l'absorption d'un phonon (Phn sur la figure 3). Cet électron se désexcite de manière radiative vers le niveau E2 en émettant un photon de longueur d'onde 1000 nm (longueur d'onde λ2 de ré-émission sur la figure 3). Un phonon est encore absorbé de manière à ce que l'électron revienne à son état original. La figure 3 illustre le principe de fonctionnement d'un tel cristal.

En variante de réalisation, le cristal employé pourra fonctionner selon l'effet Brillouin. Comme la fluorescence "anti Stokes" évoquée ci-dessus, ce phénomène est également une diffusion inélastique dans un cristal éclairé par un laser. La seule différence réside dans le fait que le décalage en longueur d'onde est plus faible et dépendant de la longueur d'onde du laser d'excitation.

Dans un premier mode de réalisation représenté sur la figure 2A, l'élément de refroidissement 11 comporte un échangeur thermique 111 reliant son doigt froid 110 à l'ensemble formé du cristal et de la cavité et permettant de transmettre la variation de température subie par le cristal vers le doigt froid.

L'échangeur thermique est choisi de manière à minimiser le gradient de température produit entre le cristal et le doigt froid. A titre d'exemple, l'échangeur thermique 111 est réalisé en saphir. De manière avantageuse un traitement diélectrique est réalisé à la jonction entre le cristal 13 et l'échangeur thermique 111 en saphir.

Avantageusement, l'élément de refroidissement 11 comporte un miroir plan-concave 112 agencé entre l'échangeur thermique et le cristal et destiné à réfléchir le rayonnement incident sur le cristal. Ce miroir est traité HR (hautement réfléchissant) dans la gamme des longueurs d'onde visibles [400 - 700] et IR [900 - 1100].

Les éléments formés du doigt froid 110, de l'échangeur thermique 111 et de la cavité 12 enfermant le cristal 13 sont positionnés de manière adjacente de manière à être en contact les uns avec les autres selon l'agencement décrit ci-dessus. La matière de la tige formant le surmoulage ne vient donc pas interférer entre ces éléments pour ne pas perturber le fonctionnement du dispositif.

Selon une variante de réalisation représentée sur la figure 2B, l'élément de refroidissement 11 est réalisé sous la forme d'un élément monobloc dans lequel est réalisée ladite cavité enfermant le cristal de manière hermétique. L'élément de refroidissement 11 remplit donc à la fois le rôle d'échangeur thermique et celui de doigt froid. L'élément de refroidissement 11 est alors préférentiellement fabriqué en saphir ou en titane. Comme décrit précédemment, il est recouvert d'un surmoulage en silicone. Il intègre également le miroir plan concave défini ci-dessus, ce miroir formant alors une paroi interne de la cavité 12 qui loge le cristal 13.

Comme décrit précédemment, le cristal 13 est amené à générer du froid lorsqu'il est illuminé pas un signal lumineux incident. Pour cela, le dispositif comporte au moins un premier guide optique 15 qui s'étend le long de la tige de son extrémité proximale jusqu'au cristal située à son extrémité distale. Le guide optique 15 est agencé pour déboucher dans la cavité 12 dans laquelle est logé le cristal 13 en vue de pouvoir véhiculer une lumière jusque dans ladite cavité 12. Le signal lumineux est généré à une longueur d'onde déterminée pour exciter le cristal.

Selon l'invention, pour évacuer la lumière produite par le cristal 13 par fluorescence, plusieurs options sont envisageables :
- Laisser la lumière produite se diffuser dans le tissu biologique (elle se situe dans la fenêtre d'absorption minimale 700 nm à 1000 nm). Elle finira ainsi par être absorbée (absorption de 0.1 à 0.5 cm-1) loin du cristal sans créer d'échauffement délétère.
- Piéger la lumière produite dans la cavité où se trouve le cristal puis l'évacuer par des canaux de fibre optique afférents. Dans cette configuration, le dispositif comporte avantageusement, en parallèle du premier guide optique, un ou plusieurs autres guides optiques 19 destinés à évacuer ou à surveiller le niveau de lumière générée par le cristal par fluorescence lors de l'excitation. Sur les figures 1 et 2, un seul guide optique "de retour" est représenté mais il faut comprendre que plusieurs guides supplémentaires peuvent être employés.

Selon la configuration employée les parois internes et externes de la cavité seront traités de manières différentes :
- Traitement HR dans le visible, et
- Traitement HT dans l'infrarouge, si absence guide optique d'évacuation, ou
- Traitement HR dans l'infrarouge si présence de guide optique d'évacuation.

Pour générer le signal lumineux d'excitation à travers le premier guide optique, le dispositif intègre avantageusement un système d'illumination 17. Le système d'illumination 17 comporte une source S de lumière employée pour émettre le signal lumineux d'excitation à destination du cristal 13 à travers le guide optique 15. La source S pourra être de différents types, par exemple formée :
- d'une diode Laser,
- d'un Laser à fibre,
- d'un Laser de type DPSS ("Diode-Pumped Solid State") ou,
- d'un Laser de type OPS ("Optically-Pumped Semiconductor").

Avantageusement, la sortie de la source S viendra se connecter directement sur le guide optique 15 du dispositif 1.

Le système d'illumination 17 comporte bien entendu une alimentation électrique 16 préférentiellement logée dans un boîtier hermétique. Selon deux variantes distinctes de réalisation, la source S lumineuse du système d'illumination est intégrée audit boîtier ou déportée par rapport à ce boîtier. Dans le premier cas, la source S est couplée au guide optique 15 par l'intermédiaire d'un connecteur démontable.

Comme décrit ci-dessus, le système d'illumination 17 est commandé par des moyens de commande de l'unité de commande et de traitement UC. Ces moyens de commande sont destinés à générer un signal de commande S2 vers le système d'illumination lorsqu'une crise est détectée. L'unité de commande et de traitement UC est avantageusement logée dans ledit boîtier hermétique et alimentée par ladite alimentation électrique 16.

De manière non limitative, l'alimentation électrique 16 du dispositif 1 pourra comporter une batterie rechargeable, une pile électrique ou employer une solution de génération d'énergie, par exemple à l'aide d'un générateur électromagnétique qui comporte une antenne destinée à générer un courant électrique induit. Toute autre solution pourrait bien entendu être envisagée comme un générateur d'énergie par vibration mécanique ou un générateur thermoélectrique.

Le dispositif de refroidissement 1 intègre préférentiellement un capteur de température 18 pour déterminer la température du cristal 13. Les données de températures mesurées par le capteur sont avantageusement envoyées vers l'unité de commande et de traitement UC en vue de contrôler en temps réel le niveau de refroidissement appliqué par le dispositif de l'invention et de le réguler si nécessaire en exécutant une boucle de régulation de température.

Le capteur de température employé utilise par exemple une technologie par mesure optique. La figure 4 illustre le principe de fonctionnement d'un tel capteur. Le système d'illumination 17 comporte un miroir dichroïque 170 placé sur le trajet optique de la lumière émise par la source S. Celle-ci est partiellement renvoyé vers le capteur 18 à travers un filtre pour analyse et détermination de la température correspondante.

Une solution optique particulière consiste à employer des fibres à réseau de Bragg distribué, afin de mesurer la longueur d'onde du signal lumineux émis et d'en déduire la température du cristal 13. Cette solution est bien connue dans l'état de la technique.

En variante de réalisation, le capteur de température employé est un thermocouple, soit une PT100 ou PT1000, déposé en couche mince et intégré à l'échangeur thermique.

Pour une application de traitement de cancer, une variante de réalisation du dispositif de l'invention consiste à intégrer à l'extrémité de la tige un système pouvant être installé dans la cavité d'exérèse pour se conformer au mieux à la forme de cette dernière. Il s'agira par exemple d'un ballonnet gonflable avec du liquide, tel que du sérum physiologique. La forme de l'échangeur thermique sera également adaptée pour se conformer au mieux à la forme de la tumeur (forme déterminée sur imagerie en préopératoire). Pour cette application le dispositif de refroidissement peut fonctionner en continu, sans employer de moyens de détection 20 tels que décrits ci-dessus.

Avantageusement, les moyens de détection 20 décrits ci-dessus pourront être intégrés au dispositif de refroidissement de l'invention. Ceux-ci seront par exemple positionnés à l'extrémité distale de la tige à proximité du doigt froid. Le dispositif 1 comportera alors à la fois les fonctions de détection et de traitement par refroidissement localisé.

De manière générale, le dispositif de refroidissement 1 présente ainsi les particularités suivantes :
- Souplesse pour éviter de dégrader les tissus de l'être vivant ;
- Présence d'une extrémité distale de forme arrondie ou oblongue pour faciliter la pénétration dans les tissus et éviter de les léser ;
- Propriété anti-écrasement pour éviter la rupture du guide d'onde pendant l'intervention chirurgicale ;
- L'élément de refroidissement définit une zone de refroidissement de surface adaptée à la zone à traiter ;

En utilisant un cristal de Yb :YLF dopé entre 5% et 10% de longueur 5 à 12 mm nous pouvons espérer obtenir un rendement de refroidissement de 1 à 2%. En fonction de la puissance du laser d'excitation nous pourrions donc obtenir des puissances de refroidissement de quelques mW à quelques centaines de mW.

## Revendications

1. Dispositif de refroidissement (1) localisé d'un organe, **caractérisé en ce qu'**il comporte :
- Une tige (10) de forme allongée comportant une extrémité distale destinée à venir en contact avec un organe à refroidir et comportant :
∘ Un élément de refroidissement (11) comportant un doigt froid (110),
∘ Un cristal (13) à capacité de refroidissement par excitation à une longueur d'onde d'excitation déterminée, positionné de manière adjacente audit élément de refroidissement,
∘ Un guide optique (15) apte à véhiculer un signal lumineux à ladite longueur d'onde d'excitation et débouchant sur ledit cristal (13),
- Un système d'illumination (17) comportant au moins une source (S) de lumière agencée pour émettre ledit signal lumineux.

2. Dispositif selon la revendication 1, **caractérisé en ce qu'**il comporte une cavité (12) dans laquelle est logé ledit cristal (13).

3. Dispositif selon la revendication 2, **caractérisé en ce que** l'élément de refroidissement (11) comporte un échangeur thermique (111) agencé entre le doigt froid (110) et la cavité logeant le cristal (13).

4. Dispositif selon l'une des revendications 1 à 3, **caractérisé en ce qu'**il comporte au moins un deuxième guide optique (19) agencé le long la tige (10) et débouchant sur ledit cristal (13) de manière à évacuer ou surveiller le niveau de fluorescence émis par l'excitation du cristal (13).

5. Dispositif selon l'une des revendications 1 à 4, **caractérisé en ce qu'**il comporte une unité de commande et de traitement (UC) agencée pour commander ledit système d'illumination.

6. Dispositif de refroidissement selon la revendication 5, **caractérisé en ce qu'**il comporte un capteur de température (18) agencé pour déterminer la température du cristal (13).

7. Dispositif de refroidissement selon la revendication 6, **caractérisé en ce que** ledit capteur de température (18) comporte des moyens de détermination de la température du cristal à partir de la longueur d'onde du signal lumineux émis par la source (S) de lumière.

8. Dispositif selon l'une des revendications 1 à 7, **caractérisé en ce que** la tige (10) est formée d'un surmoulage en silicone ou polyuréthane.

9. Dispositif selon l'une des revendications 1 à 8, **caractérisé en ce que** le cristal est de type Yb:YLF, Zb:LANP ou Tm:Yb:YLF.

10. Dispositif selon l'une des revendications 1 à 9, **caractérisé en ce que** la source (S) de lumière est choisie parmi :
- une diode Laser,
- un Laser à fibre,
- un Laser de type DPSS ("Diode-Pumped Solid State"), ou
- un Laser de type OPS ("Optically-Pumped Semiconductor").

## Patentansprüche

1. Vorrichtung zur örtlich begrenzten Kühlung (1) eines Organs, **dadurch gekennzeichnet, dass** sie Folgendes aufweist:
- Einen Stab (10) mit länglicher Form, der ein distales Ende aufweist, das dazu bestimmt ist, mit einem Organ in Kontakt zu treten, das gekühlt werden soll, und aufweisend:
∘ Ein Kühlelement (11), das einen Kühlfinger (110) aufweist,
∘ Einen Kristall (13) mit Fähigkeit zur Kühlung durch Anregung auf einer bestimmten Anregungswellenlänge, das angrenzend an das Kühlelement positioniert ist,
∘ Einen optischen Leiter (15), der geeignet ist, ein Lichtsignal auf der Anregungswellenlänge zu übermitteln und der zu dem Kristall (13) führt,
- Ein Beleuchtungssystem (17), das mindestens eine Lichtquelle (S) aufweist, die angeordnet ist, das Lichtsignal abzugeben.

2. Vorrichtung nach Anspruch 1, **dadurch gekennzeichnet, dass** sie einen Hohlraum (12) aufweist, in dem der Kristall (13) aufgenommen ist.

3. Vorrichtung nach Anspruch 2, **dadurch gekennzeichnet, dass** das Kühlelement (11) einen Wärmetauscher (111) aufweist, der zwischen dem Kühlfinger (110) und dem Hohlraum angeordnet ist, der den Kristall (13) aufnimmt.

4. Vorrichtung nach einem der Ansprüche 1 bis 3, **dadurch gekennzeichnet, dass** sie mindestens einen zweiten optischen Leiter (19) aufweist, der entlang dem Stab (10) angeordnet ist und zu dem Kristall (13) führt, um das Niveau der Fluoreszenz, die durch die Anregung des Kristalls (13) abgegeben wird, abzuleiten oder zu überwachen.

5. Vorrichtung nach einem der Ansprüche 1 bis 4, **dadurch gekennzeichnet, dass** sie eine Steuerungs- und Verarbeitungseinheit (UC) aufweist, die angeordnet ist, das Beleuchtungssystem zu steuern.

6. Kühlvorrichtung nach Anspruch 5, **dadurch gekennzeichnet, dass** sie einen Temperatursensor (18) aufweist, der angeordnet ist, die Temperatur des Kristalls (13) zu bestimmen.

7. Kühlvorrichtung nach Anspruch 6, **dadurch gekennzeichnet, dass** der Temperatursensor (18) Mittel zum Bestimmen der Temperatur des Kristalls anhand der Wellenlänge des Lichtsignals aufweist, das vom der Lichtquelle (S) abgegeben wird.

8. Vorrichtung nach einem der Ansprüche 1 bis 7, **dadurch gekennzeichnet, dass** der Stab (10) von einer Überformung aus Silikon oder Polyurethan gebildet ist.

9. Vorrichtung nach einem der Ansprüche 1 bis 8, **dadurch gekennzeichnet, dass** der Kristall vom Typ Yb:YLF, Zb:LANP oder Tm:Yb:YLF ist.

10. Vorrichtung nach einem der Ansprüche 1 bis 9, **dadurch gekennzeichnet, dass** die Lichtquelle aus Folgendem ausgewählt ist:
- einer Laserdiode,
- einem Faserlaser,
- einem DPSS-Laser ("Diode-Pumped Solid State"), oder
- einem OPS-Laser ("Optically-Pumped Semiconductor").

## Claims

1. Device (1) for cooling an organ locally, **characterized in that** it comprises:
- An elongate stem (10) comprising a far end intended to make contact with an organ to be cooled and comprising:
∘ A cooling element (11) comprising a cold finger (110),
∘ A crystal (13) that has a capacity to cool via excitation at a set excitation wavelength, said crystal being positioned adjacent to said cooling element,
∘ An optical guide (15) that is able to convey a light signal at said excitation wavelength and that opens onto said crystal (13),
- An illuminating system (17) comprising at least one light source (S), which light source is arranged to emit said light signal.

2. Device according to Claim 1, **characterized in that** it comprises a cavity (12) in which said crystal (13) is housed.

3. Device according to Claim 2, **characterized in that** the cooling element (11) comprises a heat exchanger (111) that is arranged between the cold finger (110) and the cavity housing the crystal (13).

4. Device according to one of Claims 1 to 3, **characterized in that** it comprises at least one second optical guide (19) that is arranged along the stem (10) and that opens onto said crystal (13) so as to remove or monitor the fluorescence level emitted by the excitation of the crystal (13).

5. Device according to one of Claims 1 to 4, **characterized in that** it comprises a processing and control unit (UC) arranged to control said illuminating system.

6. Cooling device according to Claim 5, **characterized in that** it comprises a temperature sensor (18) arranged to determine the temperature of the crystal (13).

7. Cooling device according to Claim 6, **characterized in that** said temperature sensor (18) comprises means for determining the temperature of the crystal from the wavelength of the light signal emitted by the light source (S).

8. Device according to one of Claims 1 to 7, **characterized in that** the stem (10) is formed by an overmolding made of silicone or polyurethane.

9. Device according to one of Claims 1 to 8, **characterized in that** the crystal is of Yb:YLF, Zb:LANP or Tm:Yb:YLF type.

10. Device according to one of Claims 1 to 9, **characterized in that** the light source (S) is chosen among:
- a laser diode,
- a fiber laser,
- a DPSS (diode-pumped solid-state) laser, or
- an OPS (optically-pumped semiconductor) laser.
